**Europäisches Patentamt**

**European Patent Office**

**Office européen des brevets**

(11) Veröffentlichungsnummer: **0 346 786**
**A2**

(12)

# EUROPÄISCHE PATENTANMELDUNG

(21) Anmeldenummer: **89110546.2**

(22) Anmeldetag: **10.06.89**

(51) Int. Cl.⁴: **A61M 25/00**

(30) Priorität: **15.06.88 DE 3820348**

(43) Veröffentlichungstag der Anmeldung:
**20.12.89 Patentblatt 89/51**

(84) Benannte Vertragsstaaten:
**AT BE CH DE ES FR GB GR IT LI LU NL SE**

(71) Anmelder: **KALETO AG**
**Kolinplatz 9**
**CH-6301 Zug(CH)**

(72) Erfinder: **Brinkhaus, Bernhard**
**Baumgartenstrasse 11**
**CH-8122 Pfaffhausen(CH)**

(74) Vertreter: **Hafner, Dieter, Dr.rer.nat.,**
**Dipl.-Phys.**
**Ostendstrasse 132**
**D-8500 Nürnberg 30(DE)**

(54) **Verfahren und Vorrichtung zur Herstellung eines Gehäuses für einen i.w. aus einem Halbleiter-Chip bestehenden Sensor, sowie Katheter mit Sensorgehäuse.**

(57) Die Herstellung eines Gehäuses z. B. für einen piezoresistiven Drucksensor, wobei ein mit einer druckempfindlichen Membrane versehener Silizium-Chip in ein vorbereitetes Gehäuse in Form einer aus Edelstahl bestehenden Metallhülse von Hand eingesetzt und eingeklebt wird, ist bisher außerordentlich zeitaufwendig und daher mit relativ hohen Kosten verbunden.

Um die Produktionskosten für Sensorgehäuse, insbesondere für als Sensorgehäuse dienende Katheterspitzen von piezoresistiven Miniatur-Kathetern, zu senken, wird bei dem erfindungsgemäßen Verfahren das Sensorgehäuse unter Verwendung preisgünstiger Spritzgußmassen mittels Spritzgußverfahrens hergestellt, wobei während des Spritzgießens eine Zerstörung des den Sensor bildenden Halbleiter-Chips, z. B. Silizium-Chips, vollkommen vermieden wird.

Ein Spritzgußwerkzeug (1, 2) weist eine der gewünschten Gehäuseform entsprechende Formhöhlung (14) auf und empfindliche Bereiche der Chip-oberfläche sind durch in der Formhöhlung angeordnete Abdeckelemente (10, 12, 13) gegenüber Druckbeaufschlagung beim Spritzgießen geschützt.

Bei vorgegebener Anzahl von Formhöhlungen (14) kann gleichzeitig eine entsprechende Anzahl von Spritzgußteilen in Sensorgehäuseform mit darin integrierten Halbleiter-Chips produziert werden.

Fig. 4

## Verfahren und Vorrichtung zur Herstellung eines Gehäuses für einen i. w. aus einem Halbleiter-Chip bestehenden Sensor, sowie Katheter mit Sensorgehäuse

Die Erfindung betrifft ein Verfahren zur Herstellung eines Gehäuses für einen im wesentlichen aus einem Halbleiter-Chip, insbesondere Silizium, bestehenden Sensor, insbesondere Druck- und/oder Temperatur-Sensor, pH-Sensor oder dergleichen Sensor.

Im Speziellen bezieht sich die Erfindung auf ein Verfahren zur Herstellung eines Gehäuses für einen piezoresistiven Drucksensor, der im wesentlichen aus einem Silizium-Chip besteht, in dem eine druckempfindliche Membrane ausgebildet ist.

Ferner betrifft die Erfindung einen Katheter, insbesondere Miniatur-Katheter, Z.B. für die Anwendung in Bereichen der Kardiologie, Urologie oder Gastroenterologie, wobei dieser Katheter mit einem nach dem erfindungsgemäßen Verfahren hergestellten Gehäuse für einen piezoresistiven Drucksensor versehen ist.

Schließlich betrifft die Erfindung noch eine Vorrichtung zur Durchführung des erfindungsgemäßen Verfahrens.

Der Stand der Technik:

Ein bekanntes Verfahren zur Herstellung eines Gehäuses für einen z. B. aus einem Silizium-Chip bestehenden Sensor besteht im wesentlichen darin, daß dieser Chip, in welchem beispielsweise eine druckempfindliche Membrane durch anisotropes Ätzen hineingebracht ist, in ein vorbereitetes Gehäuse in Form einer beispielsweise aus Edelstahl bestehenden Metallhülse eingesetzt wird. Dieses hülsenförmige Gehäuse weist eine nach außen führende Bohrung oder Öffnung auf, wobei der Silizium-Chip in der Weise angeordnet wird, daß die Membrane deckungsgleich mit dieser Bohrung oder Öffnung sich befindet. Handelt es sich beispielsweise um einen piezoresistiven Drucksensor, dann dient die Öffnung oder Bohrung in der Metallhülse zur Einleitung etwa einer Flüssigkeit, deren Druck gemessen werden soll, wobei mit dem entsprechenden Flüssigkeitsdruck die druckempfindliche Membrane beaufschlagt wird. Auf der gegenüberliegenden Seite des Silizium-Chips muß ein Atmosphären-Druckkanal zur Membranenrückseite führen, damit quasi ein Referenzdruck auf der Membranenrückseite herrscht. Beim Einsetzen des Silizium-Chips in die zugehörige Metallhülse ist es weiterhin erforderlich, daß der Silizium-Chip im Bereich zwischen Druckeinlaßkanal und Membranenrückseite ausreichend abgedichtet ist, zu welchem Zweck es bekannt ist, den Silizium-Chip abdichtend im Inneren des durch die Metallhülse gebildeten Gehäuses zu verkleben, d.h. ein - oder aufzukleben, derart, daß die Membranenvorderseite deckungsgleich mit dem in der Metallhülse vorgesehenen Loch zur Erfassung des Flüssigkeitsdruckes oder dergleichen zu liegen kommt.

Dieses bekannte Verfahren zur Herstellung eines Gehäuses für einen piezoresistiven Drucksensor ist jedoch außerordentlich aufwendig und selbst eine eingearbeitete Kraft benötigt ca. einen ganzen Tag für die Herstellung einen einzigen Sensorgehäuses. Die Kosten für ein solches Gehäuse einschließlich des Sensors liegen derzeit beispielsweise bei sfr 2000, woraus folgt, daß das bekannte Herstellungsverfahren sich als außerordentlich kostspielig ausweist.

Im übrigen ist es bekannt, ein derartiges Sensorgehäuse beispielsweise in der Form eines Miniatur-Katheters zur Messung des intraarteriellen Druckes auszubilden, wobei in der Spitze dieses Miniatur-Katheters der bereits oben erläuterte piezoresistive Drucksensor eingesetzt ist. Hierdurch lassen sich die intraarteriellen Druckschwankungen, welche auf die Membrane des Silizium-Chips einwirken, sehr genau messen, da die Eigenfrequenz des piezoresistiven Drucksensors oberhalb von 100 KHz liegt.

Auf dem Silizium-Chip ist eine Wheatstonesche Brücke integriert, die beispielsweise mit einem Konstantstrom von lmA gespeist wird. Aufgrund des piezoresistiven Effektes ändern sich bei Druckeinwirkungen auf die Membrane die Brückenwiderstände, wodurch eine dem zu messenden Druck proportionale Brückenspannung erhalten wird.

Aufgrund des relativ komplizierten und insbesondere langwierigen Herstellungsverfahrens des die Katheterspitze darstellenden Sensorgehäuses sind jedoch auch diese bekannten piezoresistiven Miniatur-Katheter heutzutage außerordentlich teuer, da es sich hierbei praktisch um Spezialanfertigungen handelt.

Im Hinblick auf die dem Stand der Technik anhaftenden Nachteile liegt der vorliegenden Erfindung nun die Aufgabe zugrunde, ein verbessertes Verfahren zur Her stellung eines Gehäuses für ein im wesentlichen aus einem Halbleiter-Chip, insbesondere einem Silizium-Chip, bestehenden Sensor anzugeben, und dieses Verfahren in der Weise auszugestalten, daß die Herstellungskosten für das Sensorgehäuse drastisch gesenkt werden können.

Diese Aufgabe wird erfindungsgemäß durch den Ablauf der Verfahrensschritte a), b) und c) gemäß dem kennzeichnenden Teil des Patentanspruchs 1 gelöst.

Vorteilhafte weitere Verfahrensausgestaltungen ergeben sich aus den Ansprüchen 2 - 4.

Im Speziellen liegt nun weiterhin der Erfindung die Aufgabe zugrunde, ein Verfahren zur Herstellung eines Gehäuses für einen piezoresistiven Drucksensor in Form eines Silizium-Chips anzugeben, in dem eine druckempfindliche Membrane ausgebildet ist, somit für einen Drucksensor, wie er bereits eingangs zum Stand der Technik erläutert wurde.

Das erfindungsgemäße Verfahren zur Herstellung eines Gehäuses für einen piezoresistiven Drucksensor ist durch den Ablauf der Verfahrensschritte a) - d) in Übereinstimmung mit dem kennzeichnenden Teil des Anspruchs 5 charakterisiert.

Weitere vorteilhafte Verfahrensausgestaltungen ergeben sich aus den Ansprüchen 6 - 8, welche sich auf die Herstellung eines Gehäuses in der Form einen Katheters bzw. einer Katheterspitze beziehen.

Als Kerngedanke des erfindungsgemäßen Verfahrens wird es angesehen, das Sensorgehäuse und insbesondere das Gehäuse für einen piezoresistiven Drucksensor mittels Spritzgußverfahren herzustellen, und zwar unter Anwendung preisgünstiger Spritzgußmassen, z. B. von Polyethylen, wobei Vorsorge dafür getroffen ist, daß während des Spritzgußverfahrens eine Zerstörung des den Sensor bildenden Halbleiter-Chips, insbesondere des Silizium-Chips vollkommen vermieden wird.

Unter Anwendung des erfindungsgemäßen Verfahrens ist ferner eine Herstellung des Sensorgehäuses in einem einzigen Arbeitsgang gewährleistet, wobei gleichzeitig die Möglichkeit der Massenfertigung derartiger Sensorgehäuse gegeben ist.

Gegenüber dem Stand der Technik wird durch das erfindungsgemäße Herstellungsverfahren eine wesentliche Herabsetzung der Produktionskosten für die Sensorgehäuse und somit auch für die resultierenden Sensoren oder für komplette piezoresistive Miniatur-Katheter erreicht.

Der Erfindung liegt nun ferner die Aufgabe zugrunde, einen verbesserten und insbesondere sehr preisgünstig herstellbaren Katheter, insbesondere Miniatur-Katheter zu schaffen, welcher mit einem piezoresistiven Drucksensor versehen ist und insbesondere in den medizinischen Bereichen der Kardiologie, Urologie oder Gastroenterologie einsetzbar ist, d. h. beispielsweise auf dem Gebiet der Kardiologie zur Messung des intraarteriellen Blutdruckes.

Diese Aufgabe wird gemäß der Erfindung dadurch gelöst, daß der Katheter und insbesondere die Katheterspitze als ein Spritzgußteil mit Hilfe des wie oben schon definierten, erfindungsgemäßen Verfahrens zur Herstellung eines Gehäuses für einen piezoresistiven Drucksensor hergestellt ist und daß ein derartiger Drucksensor im Bereich der als Sensorgehäuse dienenden Katheterspitze angeordnet ist.

Schließlich liegt der vorliegenden Erfindung noch die Aufgabe zugrunde, eine Vorrichtung zur Herstellung eines Gehäuses für einen im wesentlichen aus einem Halbleiter-Chip, vorzugsweise Silizium, bestehenden Sensor, insbesondere Druck- und/oder Temperatursensor, pH-Sensor oder dergleichen anzugeben, und insbesondere eine Vorrichtung zur Durchführung des erfindungsgemäßen Verfahrens.

Eine derartige Vorrichtung ist gemäß der Erfindung durch die Merkmale des kennzeichnenden Teiles des Patentanspruches 10 charakterisiert.

Weitere vorteilhafte Ausgestaltungen der erfindungsgemäßen Vorrichtung ergeben sich. aus den Ansprüchen 11 -20.

Durch die Erfindung werden im wesentlichen die folgenden Vorteile erzielt:

1. Es ist erstmalig die Möglichkeit gegeben, auf preiswerte Art und Weise Gehäuse für Halbleiter-Sensoren auf Chip-Basis mittels Spritzgußverfahren herzustellen, wobei ein ausreichender Schutz der empfind lichen Sensoroberfläche oder empfindlicher Bereiche dieser Sensoroberfläche gegenüber Druckbeaufschlagung während des Spritzgußvorganges gewährleistet ist.

2. Es besteht die Möglichkeit, das Spritzgußwerkzeug mit einer größeren Anzahl von der gewünschten Sensorgehäuseform entsprechenden Formhöhlungen zu versehen, d. h. dieses Spritzgußwerkzeug als ein Mehrfachwerkzeug, z. B. Zehnfachwerkzeug, auszubilden, so daß eine entsprechende Anzahl von Sensorgehäusen mit Hilfe nur eines einzigen Spritzschusses sicher gefertigt werden kann, wobei reproduzierbare Verhältnisse gegeben sind.

3. Mit Hilfe des erfindungsgemäßen Verfahrens sowie der zu dessen Durchführung dienenden Vorrichtung lassen sich in einfacher Weise Kunststoffgehäuse für verschiedenartige Sensoren, wie z. B. Drucksensoren, Temperatursensoren, pH-Sensoren, NOX-Sensoren oder auch anderer Sensoren produzieren, wobei derartige Sensoren alle auf Chip-Basis (insbesondere Silizium-Chip) aufgebaut sind.

4. Es ist erstmalig möglich, die bisher nur als teuere Spezialanfertigungen erhältlichen piezoresistiven Miniatur-Katheter sehr kostengünstig zu produzieren und somit auch als Einweg-Katheter zu verwenden, so daß die bislang aufgetretenen Probleme hinsichtlich der Sterilisation bei Mehrfachanwendung von derartigen Kathetern jetzt völlig entfallen. Der erfindungsgemäße Katheter und insbesondere Miniatur-Katheter wird mit einer als preisgünstiges Spritz gußteil ausgebildeten Katheterspitze ausgestattet, in welcher der piezoresistive Drucksensor eingespritzt ist.

Zur näheren Erläuterung der Erfindung, ihrer weiteren Merkmale und Vorteile dient die nachfolgende Beschreibung von Ausführungsbeispielen in Verbindung mit der Zeichnung. Hierbei sind gleiche Teile und Elemente stets mit den gleichen Bezugsziffern bezeichnet.

Dabei zeigt:

Fig. 1a in einer teilweisen Seitenansicht und in teilweisem Schnitt eine erste Werkzeugbacke eines Spritzgußwerkzeuges;

Fig. 1b in schematischer Seitenansicht und in teilweisem Schnitt eine zweite Werkzeugbacke des Spritzgußwerkzeugs;

Fig. 2 eine schematische Seitenansicht eines Einsatzelementes für die zweite Werkzeugbacke gemäß Figur 1b;

Fig. 3 eine erste Schnittansicht durch die beiden ersten und zweiten Werkzeugbacken (gemäß den Figuren 1a und 1b), wobei sich diese Werkzeugbacken in einem zusammengefahrenen Zustand befinden und damit die Formhöhlung des Spritzgußwerkzeuges gebildet ist;

Fig. 4 eine zweite schematische Schnittansicht entsprechend der Schnittansicht gemäß Figur 3, wobei nunmehr ein Silizium-Chip in der Formhöhlung des Spritzgußwerkzeuges angeordnet ist;

Fig. 5 eine schematische Schnittansicht einer als Sensorgehäuse dienenden Katheterspitze; und

Fig. 6 eine teilweise, schematische Seitenansicht eines Katheters, welcher mit der Katheterspitze gemäß Figur 5 ausgestattet ist, wobei es sich hierbei um einen Miniatur-Katheter zur Messung des intraarteriellen Blutdruckes handelt.

Alle im vorangehenden genannten Figuren sind im vergrößerten Maßstab gezeichnet.

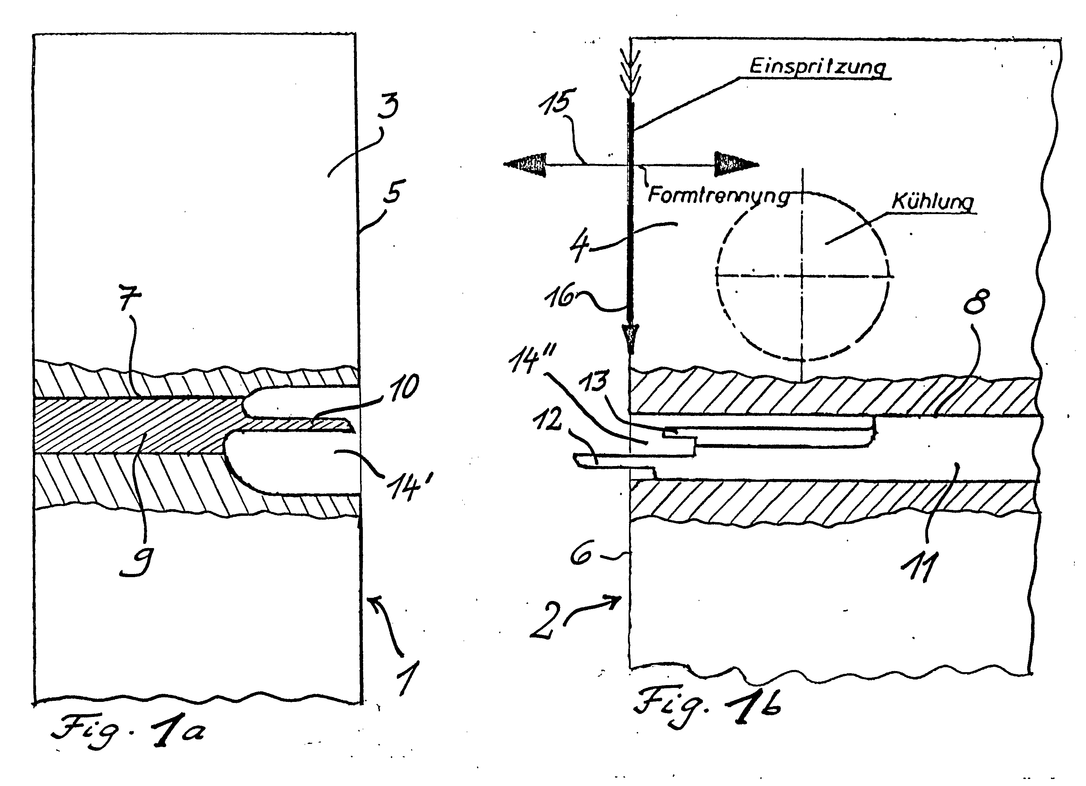

Aus den Figuren 1a und 1b gehen in einer teilweisen Seitenansicht erste und zweite Werkzeugbacken 1 und 2 eines Spritzgußwerkzeuges hervor, welches zur Herstellung eines Gehäuses für einen piezoresistiven Drucksensor in der Form eines Silizium-Chips dient.

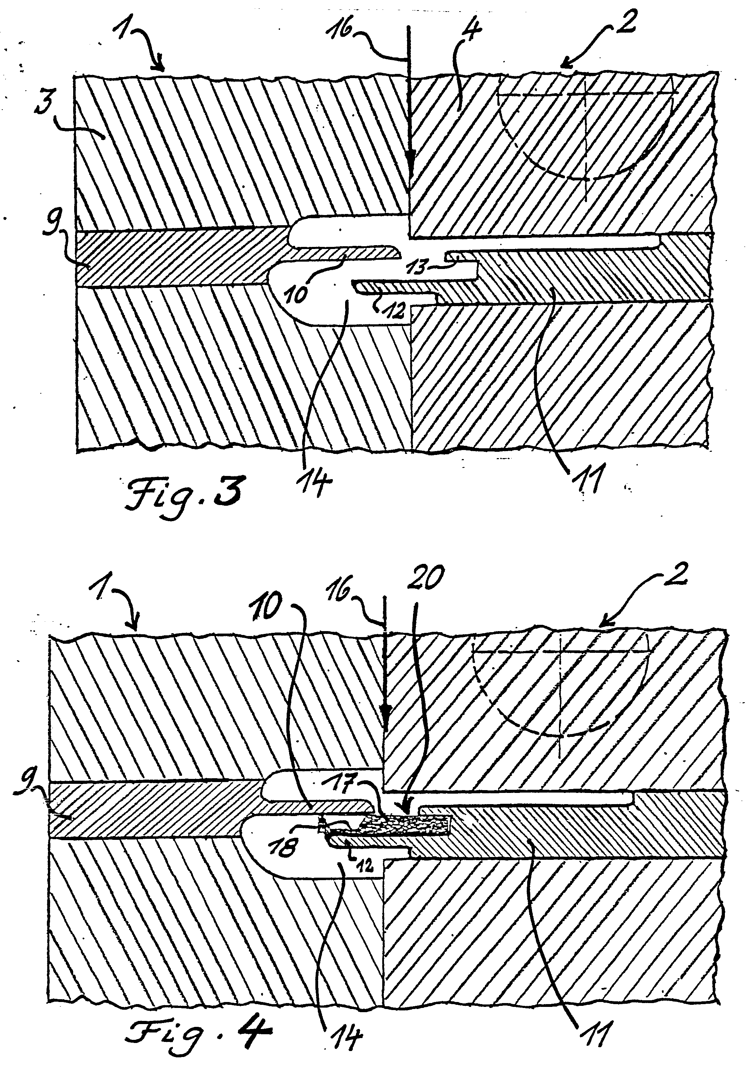

Die erste Werkzeugbacke 1 besteht im wesentlichen aus einem ersten Metallblock 3, der eine Bohrung 7 aufweist, welche zwischen zwei einander gegenüberliegenden Oberflächen des Metallblockes 3 durch diesen hindurch verläuft. Diese Bohrung 7 erweitert sich in Richtung zu einer stirnseitigen Oberfläche 5 des ersten Metallblockes 3 zu einem ersten Teilbereich 14' einer Form höhlung 14, wie diese genauer aus den Figuren 3 und 4 ersichtlich ist.

Von einer der stirnseitigen Oberfläche 5 gegenüberliegenden Oberfläche des Metallblockes 3 ist in die genannte Bohrung 7 ein Einsatzelement 9 eingesetzt. Vorzugsweise ist dieses Einsatzelement 9 von der dem ersten Teilbereich 14' der Formhöhlung abgewendeten Oberfläche des ersten Metallblockes 3 her in die Bohrung 7 einschraubbar.

Das Einsatzelement 9, welches gemäß der Ansicht nach Figur 1a bereits in den ersten Metallblock 3 vollständig eingeschraubt ist, ist im ersten Teilbereich 14' der Formhöhlung 14 mit einem ersten Abdeckelement 10 in der Form eines flachen stegartigen Vorsprunges des Einsatzelementes 9 versehen. Die Bedeutung dieses ersten Abdeckelementes 10 wird noch weiter unten erläutert.

Die aus Figur 1b ersichtliche zweite Werkzeugbacke 2 besteht im wesentlichen aus einem zweiten Metallblock 4, welcher hinsichtlich Form und Abmessungen identisch ist mit dem ersten Metallblock 3.

In gleicher Weise wie bei dem ersten Metallblock 3 ist nun auch bei dem zweiten Metallblock 4 eine sich zwischen zwei einander gegenüberliegenden Oberflächen durchgehend erstreckende Bohrung 8 ausgebildet, in welcher ein der zweiten Werkzeugbacke 2 zugeordnetes Einsatzelement 11 untergebracht ist. Dieses Einsatzelement 11 ist in Richtung zu einer stirnseitigen Oberfläche 6 des zweiten Metallblockes 4 hin gesehen in der Weise ausgebildet, daß sich unterhalb dieser stirnseitigen Oberfläche 6 ein zweiter Teilbereich 14" der Formhöhlung 14 (vgl. Figur 3, 4) ergibt. Auch bei der zweiten Werkzeugbacke 2 ist das Einsatzelement 11 von der dem zweiten Teilbereich 14" der Formhöhlung 14 abgewendeten Oberfläche des zweiten Metallblockes 4 in dessen Bohrung 8 einsetzbar und vorzugsweise einschraubbar. Figur 1b zeigt den Zustand der zweiten Werkzeugbacke 2 mit vollständig eingeschraubtem Einsatzelement 11. Hierbei ist auch ersichtlich, daß dieses Einsatzelement 11 in seinem dem zweiten Teilbereich 14" der Formhöhlung 14 zugeordneten Endabschnitt in der Weise ausgebildet ist, daß dieser Endabschnitt zweite und dritte Abdeckelemente 12 und 13 aufweist, welche ähnlich wie das erste Abdeckelement 10 des Einsatzelementes 9 im wesentlichen vorsprungartig ausgebildet sind.

Auch die Bedeutung und Funktionsweise der zweiten und dritten Abdeckelemente 12 und 13 ergibt sich aus den weiter unten noch folgenden Erläuterungen.

In der Figur 1b ist ferner noch durch einen Pfeil 16 die Richtung der Einspritzung einer Spritzgußmasse angedeutet, wobei ersichtlich ist, daß diese Einspritzrichtung im Bereich der Trennung der beiden ersten und zweiten Werkzeugbacken 1 und 2 des Spritzgußwerkzeuges bzw. der Formtrennung verläuft, wie dies durch den Doppelpfeil 15 in der Figur 1b angedeutet ist.

In dieser ist ferner schematisch angedeutet, daß durch den zweiten Metallblock 4 hindurch Kanäle zur Durchleitung eines Kühlmediums verlaufen können.

Derartige Kühlmediumkanäle können selbstverständlich auch bei dem ersten Metallblock 3 gemäß Figur 1a vorgesehen sein, wie dies im einzelnen nicht dargestellt ist.

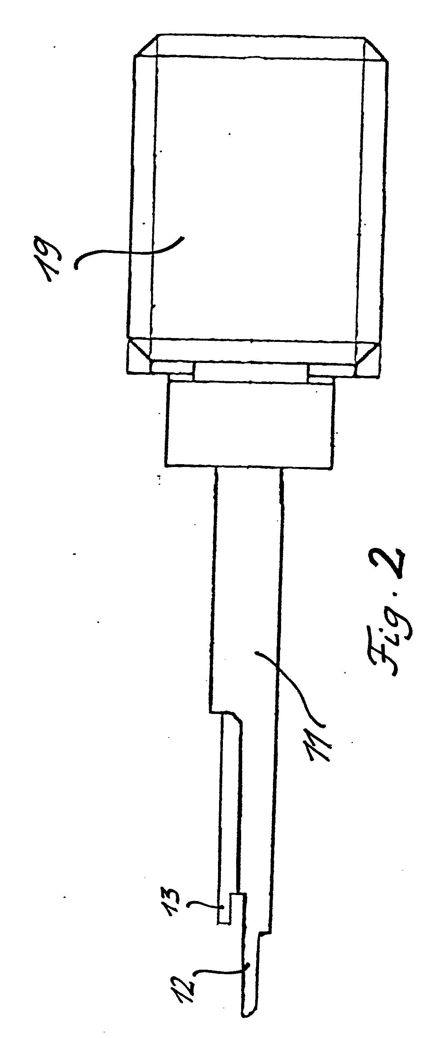

Figur 2 zeigt in schematischer Seitenansicht das vollständige Einsatzelement 11 für die zweite Werkzeugbacke 2 gemäß Figur 1b.

Das Einsatzelement 11 weist an seinem den Abdeckelementen 12 und 13 gegenüberliegenden Ende eine Schraube 19 auf, mit deren Hilfe das Einsatzelement 11 von der der stirnseitigen Oberfläche 6 gegenüberliegenden Seite des zweiten Metallblockes 4 her in die zugeordnete Bohrung 8 einschraubbar ist, wie dies der Einfachheit halber in den Zeichnungen nicht im Detail dargestellt ist. Auch das Einsatzelement 9 für die erste Werkzeugbacke 1 kann eine entsprechende Schriaube zum Einschrauben in die Bohrung 7 des ersten Metallblockes 3 aufweisen.

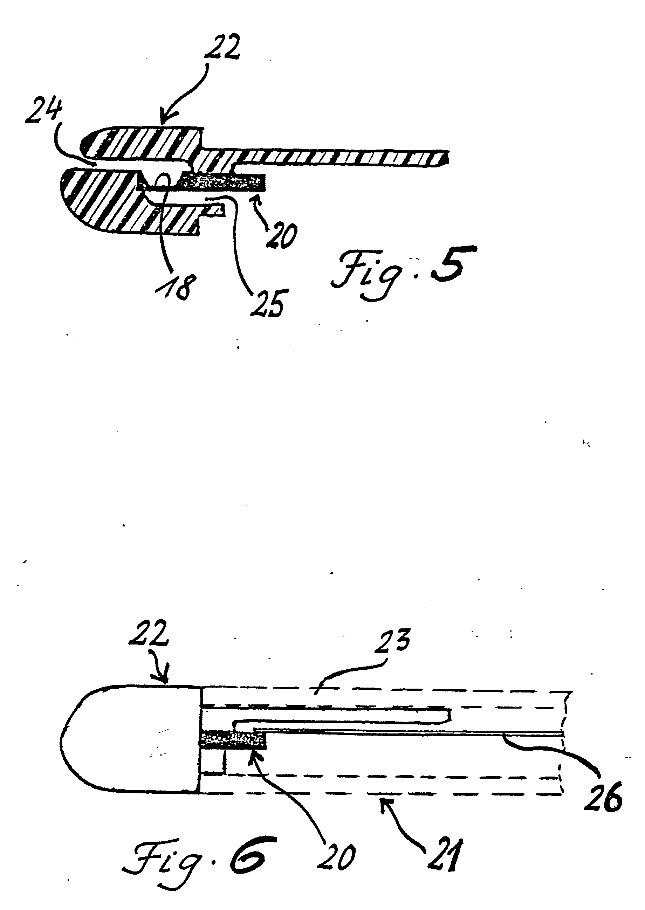

Aus den Figuren 3 und 4 ist der geschlossene Zustand der beiden ersten und zweiten Werkzeugbacken 1 und 2 ersichtlich, derart, daß die der gewünschten Gehäuseform entsprechende Formhöhlung 14 gebildet ist. Wie insbesondere Figur 3 zeigt, ist die Gestalt dieser Formhöhlung 14 insbesondere durch das Vorhandensein des ersten Abdeckelementes 10 des Einsatzelementes 9 sowie der zweiten und dritten Abdeckelemente 12 und 13 des Einsatzelementes 11 charakterisiert, wobei in dem hier erläuterten Ausführungsbeispiel eine derartige Ausbildung und Anordnung der drei Abdeckelemente 10, 12 und 13 vorgesehen ist, daß sich in Verbindung mit den in den ersten und zweiten Metallblöcken 3 und 4 weiterhin vor gesehenen Ausnehmungen im Bereich der beiden ersten und zweiten Formhöhlung-Teilbereiche 14′ und 14″ eine resultierende Gestaltung der Formhöhlung 14 ergibt, die somit den Konturen einer Spitze 22 eines Katheters 21 ensprechend ausgeformt ist, wie dies auch beispielsweise aus den Figuren 5 und 6 ersichtlich ist.

Im zusammengefahrenen oder geschlossenen Zustand der beiden ersten und zweiten Werkzeugbacken 1 und 2 bzw. der beiden ersten und zweiten Metallblöcke 3 und 4 liegen deren stirnseitigen Oberflächen 5 und 6 dicht aufeinander, wobei jedoch, wie bereits erwähnt und durch den Pfeil 16 angedeutet, Einspritzkanäle oder Einspritzdüsen zum Einspritzen der Spritzgußmassen in die Formhöhlung 14 im Bereich der aneinander angrenzenden, stirnseitigen Oberflächen 5 und 6 angeordnet und ausgebildet sind, derart, daß diese Einspritzkanäle oder-Düsen praktisch im Bereich der Werkzeugbackentrennung bzw. der Formtrennung verlaufen.

Bevor jedoch die beiden ersten und zweiten Werkzeugbacken 1 und 2 zur Bildung der Formhöhlung 14 geschlossen werden, wird ein Silizium-Chip 17 unmittelbar oberhalb des zweiten Abdeckelementes 12 und teilweise unterhalb des dritten Abdeckelemtes 13 des Einsatzelementes 11 gemäß Figur 1b bzw. Figur 2 angeordnet, wobei sich die Lage des Silizium-Chips 17 innerhalb der Formhöhlung 14 aus der Figur 4 ergibt. Hieraus ist auch ersichtlich, daß im geschlossenen Zustand der beiden Werkzeugbacken 1 und 2 das erste Abdeckelement 10 des Einsatzelementes 9 sich in einer solchen Lage befindet, daß der Silizium-Chip 17 sich im Bereich zwischen dem ersten Abdeckelement 10 und dem zweiten Abdeckelement 12 befindet. Ferner zeigt die Figur 4, daß der Silizium-Chip 17 mit einer druckempfindlichen Membrane 18 versehen ist, die insbesondere durch anisotropes Ätzen in den Silizium-Chip 17 hingebracht worden ist, derart, daß im Fall des hier erläuterten intraarteriell einführbaren Miniatur-Katheters die intraarteriellen Druckschwankungen unnmittelbar mit Hilfe des als piezoresistiver Drucksensor ausgebildeten Silizium-Chips 17 gemessen werden können, wenn dieser, wie Figuren 5 und 6 zeigen, sich in der Katheterspitze 22 des intraarteriell einführbaren Katheters 22 befindet.

Aus der Figur 4 jedenfalls ist nun eindeutig ersichtlich, daß durch die in die Formhöhlung 14 hineinragenden ersten, zweiten und dritten Abdeckelemente 10, 12 und 13 insbesondere die empfindlichen Bereiche der Oberfläche des Silizium-Chips 17, vor allem aber der Bereich der druckempfindlichen Membrane 18, gegenüber einer Druckbeaufschlagung durch die in die Formhöhlung 14 eingespritzte Spritzgußmasse sehr gut geschützt sind, so daß während des Spritzgußvorganges keinerlei Beschädigungen des Silizium-Chips 17 auftreten können.

Wie bereits weiter oben erwähnt, wird zunächst dieser Silizium-Chip 17 in den zweiten Teilbereich 14″ der Formhöhlung 14 (vgl.Fig.1b) eingesetzt, wobei also die zweiten und dritten Abdeckelemente 12 und 13 gleichzeitig als Halte- und Trägerelemente für den Silizium-Chip 17 dienen, und sodann werden die beiden ersten und zweiten Werkzeugbacken 1 und 2 langsam aufeinander zugefahren, und zwar mit Hilfe von in den Zeichnungen der Einfachheit halber nicht dargestellten Verschiebe- und Gleitmechanismen für die beiden Werkzeugbacken 1 und 2.

Mit Hilfe derartiger Verschiebe- und Gleitmechanismen können jedoch die ersten und zweiten Werkzeugbacken 1 und 2 in der Weise relativ zueinander verschiebbar im Spritzgußwerkzeug angeordnet werden, daß die einander gegenüberliegenden, stirnseitigen Oberflächen 5 und 6 der beiden Werkzeugbacken 1 und 2 in vorgegebener Richtung (vgl. Doppelpfeil 15 gemäß Figur 1b) aufeinander zufahrbar bzw. in entgegengesetzter Richtung voneinander trennbar sind, wobei in dem aufeinander zugefahrenen Zustand bis zu der gegenseitigen Kontaktgabe der stirnseitigen Oberflächen 5 und 6, wie dieser Zustand aus den Figuren 3 und 4 zu ersehen ist, die erste Werkzeugbacke 1 relativ zur zweiten Werkzeugbacke 2 in einer zur Zusammenfahrrichtung im wesentlichen senkrechten Richtung vorzugsweise um einige Mikrometer verschiebbar ist (also praktisch in der Richtung des Pfeiles 16), derart, daß sich das Abdeckelement 10 in entsprechender Weise in Richtung auf das Abdeckelement 12 hin bewegt, wodurch sichergestellt ist, daß der dazwischen gesetzte Silizium-Chip 17 sicher und gut geschützt zwischen dem ersten Abdeckelement 10 und dem zweiten Abdeckelement 12 angeordnet ist. Mit anderen Worten, durch diese Maßnahme wird dafür gesorgt, daß insbesondere der Bereich der druckempfindlichen Membrane 18 durch die Abdeckelemente 10 und 12 in ausreichendem Maße abgedeckt und abgedichtet wird, um eine Beeinträchtigung durch die unter Druck einzuleitende Spritzgußmasse von vorneherein völlig zu vermeiden.

Hierbei wird jedoch ferner sichergestellt, daß durch das erste Abdeckelement 10 ein nur minimaler Druck auf die Oberfläche des Silizium-Chips 17 oberhalb der Membrane 18 ausgeübt wird.

Wie bereits weiter oben erwähnt, stellt der im Verlauf des weiteren Spritzgußverfahrens innerhalb der Formhöhlung 14 einzuspritzende Silizium-Chip 17 mit seiner druckempfindlichen Membrane 18 einen Sensor 20 in der Art eines piezoresistiven Drucksensors dar, wie er an sich bekannt ist.

Somit läßt sich zusammenfassend feststellen, daß im zusammengefahrenen, d.h. geschlossenen Zustand der ersten und zweiten Werkzeugbacken 1 und 2, in welchem Zustand die drei Abdeckelemente 10,12 und 13 in die resultierende Formhöhlung 14 hineinragen, gewährleistet ist, daß einerseits durch das erste Abdeckelement 10 teilweise die obere Oberfläche des Silizium-Chips 17 und damit auch die Vorderseite der druckempfindlichen Membrane 18 und andererseits durch das zweite Abdeckelement 12 die untere Oberfläche des Silizium-Chips 17 und damit die druckempfindliche Membranenrückseite gegenüber Druckbeaufschlagungen während des Spritzgußvorganges geschützt sind.

Darüber hinaus ist aber auch eine derartige Ausbildung des ersten Abdeckelementes 10 des Einsatzelementes 9 vorgesehen, daß in dem resultierenden Sensorgehäuse, d. h. also nachdem das Abdeckelement 10 nach dem Erstarren der Spritzgußmasse wieder entfernt ist, ein Flüssigkeitskanal mit einer nach außen mündenden Öffnung ausgebildet ist, im Falle des Katheters also beispielsweise ein Bluteinlaßkanal 24 (vgl. Figur 5), welcher andererseits auf der Vorderseite der druckempfindlichen Membrane 18 ausmündet. Weiterhin sind die zweiten und dritten Abdeckelemente 12 und 13 des Einsatzelementes 11 in der Weise ausgebildet, daß in dem resultierenden Sensorgehäuse ein Atmosphärendruckkanal 25 als Referenzkanal ausgebildet wird (vgl. Figur 5), der zur Rückseite der druckempfindlichen Membrane 18 des Sensor 20 führt, so daß also diese Membranenrückseite dem normalen Atmosphärendruck zugänglich ist.

Es ist noch zu erwähnen, daß die beiden Einsatzelemente 9 bzw. 11 der ersten Werkzeugbacke 1 bzw. der zweiten Werkzeugbacke 2 jeweils als erodierte Edelstahl-Einsätze mit hochpolierten Oberflächen im Bereich der Formhöhlung 14 ausgebildet sind, d. h. mit hochpolierten Oberflächen an den zugeordneten Abdeckelementen 10, 12 und 13, wodurch sehr gute Oberflächen des resultierenden Spritzgußteiles gewährleistet sind und insbesondere beim Abziehen der Abdeckelemente von dem resultierenden Spritzgußteil es zu keinerlei Beschädigungen an dessen Oberflächen kommt.

Nach Einleitung der Spritzgußmasse in die Formhöhlung 14 gemäß Figur 4 und nach dem Erstarren dieser Spritzgußmasse, beispielsweise Polyethylen, oder eines ähnlichen thermoplastischen Kunststoffes, welcher primär unter Druck eingeleitet wird, wird schließlich das fertige Gehäuse in der Form der Katheterspitze 22 gemäß Figur 5 mit dem darin eingespritzten Sensor 20 aus der Formhöhlung 14 entnommen, indem die beiden Werkzeugbacken 1 und 2 auseinandergefahren und hierbei die Abdeckelemente 10,12 und 13 von den Oberflächen des Silizium-Chips 17 wieder entfernt werden, sowie auch von Oberflächenbereichen des fertigen Sensorgehäuses.

Das resultierende Spritzgußteil ist, wie bereits erwähnt, in der Figur 5 in Form einer Schnittansicht zu sehen.

Bei diesem Endprodukt handelt es sich um die Katheterspitze 22 für einen Katheter 21 gemäß Figur 6, wobei der Sensor 20 mit der druckempfindlichen Membrane 18 im Bereich der als Sensorgehäuse dienenden Katheterspitze 22 angeordnet ist. Von der frontseitigen Öffnung der Katheterspitze 22 führt der Bluteinlaßkanal 24 zur oberen Oberfläche der Membrane 18, während zu der gegenüberliegenden, unteren Oberfläche der Membrane 18 der Atmophärendruckkanal 25 führt.

Aus Figur 6 schließlich ist noch zu ersehen, daß die Katheterspitze 22 auf ihrem dem Bluteinlaßkanal 24 abgewendeten Bereich an einen Katheterschlauch 23 angeschlossen ist, welcher auf den rückwärtigen Abschnitt der Katheterspitze 22 bündig aufgeschoben ist. Hierdurch wird ein Katheterhohlraum innerhalb des Katheterschlauches 23 gebildet, in welchen eine flexible Leiterplatte (Flexprint) 26 hineinführt, welche einerseits mit der integrierten Schaltung des Silizium-Chips 17 des piezoresistiven Druck-Sensors 20 verbunden ist, während der Flexprint 26 andererseits zu den (hier nicht dargestellten) anschließenden elektronischen Meß- und Auswertegeräten führt, in denen das durch den Sensor erzeugte, druckproportionale elektrische Signal weiter verarbeitet wird.

Wie bereits weiter oben erwähnt, kann das im Rahmen des Ausführungsbeispiels erläuterte Spritzgußwerkzeug auch so ausgebildet sein, daß es eine vorgegebene Anzahl von Formhöhlungen aufweist, wobei eine entsprechende Anzahl von Bohrungen mit Einsatzelementen vorgesehen ist, so daß gleichzeitig eine entsprechende Anzahl von Spritzgußteilen in Form der Sensorgehäuse mit Halbleiter-Chip produziert werden können.

## Ansprüche

1. Verfahren zur Herstellung eines Gehäuses für einen i.w. aus einem Halbleiter-Chip, insbesondere Silizium, bestehenden Sensor, insbesondere Druck-und/oder Temperatur-Sensor, pH-Sensor oder dergleichen Sensor,
gekennzeichnet durch den Ablauf der folgenden Verfahrensschritte:

a) Der Sensor wird in eine der gewünschten Gehäuseform entsprechende Formhöhlung eines Spritzgußwerkzeuges eingebracht, wobei die Sensoroberfläche bzw. im wesentlichen die empfindlichen Bereiche der Sensoroberfläche mittels entsprechender Abdeckelemente gegenüber Druck beaufschlagung beim Spritzgießen geschützt werden;

b) in den noch verbleibenden freien Hohlraum der Formhöhlung wird sodann eine Spritzgußmasse unter Druck eingeleitet; und

c) nach Erstarren der Spritzgußmasse wird das fertige Gehäuse mit dem Sensor aus der Form entnommen, wobei die Abdeckelemente von der Sensoroberfläche entfernt werden.

2. Verfahren nach Anspruch 1,
dadurch gekennzeichnet,
daß die den Sensor während des Spritzgießens gegenüber Druckbeaufschlagung schützenden Abdeckelemente zumindest teilweise gleichzeitig als Halte-/Trägerelemente für den Sensor verwendet werden.

3. Verfahren nach Anspruch 1 oder 2,
dadurch gekennzeichnet,
daß die den Sensor während des Spritzgießens gegenüber Druckbeaufschlagung schützenden Abdeckelemente zumindest teilweise dazu verwendet werden, einen oder mehrere Kanäle in dem Gehäuse auszubilden, die insbesondere zu vorgegebenen Oberflächenbereichen des Sensors führen, zum Beispiel einen Flüssigkeitseinlaßkanal, einen Atmosphärendruckkanal oder dergleichen.

4. Verfahren nach einem der vorhergehenden Ansprüche,
dadurch gekennzeichnet,
daß gleichzeitig vom Sensor abgehende elektrische Anschluß-/Verbindungs-Leitungen in vorgegebenen Wandungsabschnitten des Gehäuses eingespritzt werden.

5. Verfahren zur Herstellung eines Gehäuses für einen piezoresistiven Drucksensor, (ggf. auch Temperatur-Sensor), der i. w. aus einem Silizium-Chip besteht, in dem eine druckempfindliche Membrane ausgebildet ist, nach einem der Ansprüche 1 - 4,
gekennzeichnet durch den Ablauf der folgenden Verfahrensschritte:

a) Der Silizium-Chip wird mit seiner unteren, die Membranenrückseite aufweisenden Oberfläche auf ein als erstes Abdeckelement dienendes Trägerelement gelegt, das innerhalb der Formhöhlung des Spritzgußwerkzeuges angeordnet und in der Weise ausgebildet ist, daß die untere Oberfläche des Silizium-Chips und insbesondere die Membranenrückseite gegenüber Druckbeaufschlagung während des Spritzgußvorganges geschützt wird;

b) sodann wird zumindest auf einen Teil der oberen Oberfläche des Silizium-Chips ein zweites Abdeckelement aufgebracht, das in der Weise ausgebildet ist, daß insbesondere der die Membranenvorderseite aufweisende Bereich des Silizium-Chips gegenüber Druckbeaufschlagung während des Spritzgußvorganges geschützt wird;

c) sodann wird in den noch verbleibenden, die Anordnung von Silizium-Chip mit den beiden ersten und zweiten Abdeckelementen umgebenden Hohlraum der Formhöhlung eine Spritzgußmasse unter Druck eingeleitet;
und

d) schließlich wird nach dem Erstarren der Spritzgußmasse das den Silizium-Chip enthaltende Gehäuse aus dem Spritzgußwerkzeug entnommen, wobei die ersten und zweiten Abdeckelemente aus dem Gehäuse entfernt werden, wodurch an der Gehäusevorderseite eine erste Öffnung mit anschließendem, zur Membranenvorderseite führenden ersten Kanal und an der Gehäuserückseite eine zweite Öffnung mit anschließendem, zur Membranenrückseite führenden zweiten Kanal gebildet werden.

6. Verfahren nach Anspruch 5,
dadurch gekennzeichnet,
daß der Silizium-Chip in ein Spritzgußwerkzeug eingebracht wird, dessen Formhöhlung den Konturen der Spitze eines Katheters entsprechend ausgeformt ist, derart, daß diese Katheterspitze i.w. das Sensor-Gehäuse bildet.

7. Verfahren nach Anspruch 6,
dadurch gekennzeichnet,
daß in der Spitze des Katheters eine frontseitige Öffnung mit anschließendem, auf der Membranenvorderseite des Silizium-Chips ausmündendem Flüssigkeitskanal ausgebildet wird.

8. Verfahren nach Anspruch 6 oder 7,
dadurch gekennzeichnet,
daß der frontseitigen Öffnung der Katheterspitze gegenüberliegend ein zur Membranenrückseite des Silizium-Chips führender Atmosphärendruckkanal ausgebildet wird.

9. Katheter, insbesondere Miniatur-Katheter, zum Beispiel für die Anwendung insbesondere im Bereich der Kardiologie, Urologie oder Gastroenterologie, mit einem piezoresistiven Drucksensor,
dadurch gekennzeichnet,
daß der Katheter, insbesondere die Katheterspitze als Spritzgußteil mittels Verfahren gemäß einem der Ansprüche 5 bis 8 hergestellt ist und der Drucksensor (20) im Bereich der als Sensorgehäuse dienenden Katheterspitze (22)angeordnet ist.

10. Vorrichtung zur Herstellung eines Gehäuses für einen im wesentlichen aus einem Halbleiter-Chip, vorzugs weise Silizium bestehenden Sensor, insbesondere Druck- und/oder Temperatursensor, pH-Sensor oder dergleichen, insbesondere zur Durchführung des Verfahrens nach einem der Ansprüche 1 - 8,
gekennzeichnet durch
ein Spritzgußwerkzeug, das eine erste Werkzeugbacke (1) und eine zweite Werkzeugbacke (2) aufweist, durch welche im geschlossenen Zustand der beiden Werkzeugbacken wenigstens eine der gewünschten Gehäuseform entsprechende Formhöhlung(14) gebildet ist, wobei für eine jede der beiden ersten und zweiten Werkzeugbacken (1) bzw. (2) Einsatzelemente (9) bzw. (11) vorgesehen sind, die im Bereich der Formhöhlung(en) (14) in der Weise ausgebildet sind, daß sie jeweils Abdeckelemente (10) bzw. (12, 13) aufweisen, durch welche die Sensoroberfläche oder im wesentlichen die empfindlichen Bereiche der Sensoroberfläche gegenüber Druckbeaufschlagung beim Spritzgießen geschützt sind.

11. Vorrichtung nach Anspruch 10,
dadurch gekennzeichnet,
daß die Abdeckelemente (12, 13) des wenigstens einen Einsatzelementes (11) für die zweite Werkzeugbacke (2) gleichzeitig als Halte- und/oder Trägerelemente für den in die Formhöhlung (14) einbegrachten Sensor ausgebildet sind.

12. Vorrichtung nach Anspruch 10 oder 11,
dadurch gekennzeichnet,
daß zumindest eines der Abdeckelemente (12,13) des Einsatzelementes (11) für die zweite Werkzeugbacke (2) gleichzeitig in der Weise ausgebildet ist, daß in dem als Spritzgußteil zu fertigenden Gehäuse wenigstens ein erster Kanal ausgebildet ist, der beispielsweise als Atmosphärendruckkanal zu einem vorgegebenen Oberflächenbereich des Sensors führt.

13. Vorrichtung nach einem der Ansprüche 10 - 12
dadurch gekennzeichnet,
daß das Abdeckelement (10) des wenigstens einen Einsatzelementes (9) für die erste Werkzeugbacke (1) gleichzeitig in der Weise ausgebildet ist, daß in dem als Spritzgußteil zu fertigenden Gehäuse wenigstens ein zweiter Kanal ausgebildet ist, beispielsweise ein Flüssigkeitskanal, der zu einem vorgegebenen Oberflächenbereich des Sensors führt.

14. Vorrichtung nach einem der Ansprüche 10 - 13, insbesondere zur Herstellung eines Gehäuses für einen piezoresistiven Drucksensor,
dadurch gekennzeichnet,
daß das Abdeckelement (12) des wenigstens einen Einsatzelementes (11) der zweiten Werkzeugbacke (2) in der Weise ausgebildet ist, daß i.w.die untere Oberfläche des den Drucksensor bildenden Silizium-Chips und damit die druckempfindliche Membranenrückseite gegenüber Druckbeaufschlagung während des Spritzgußvorganges geschützt ist.

15. Vorrichtung nach einem der Ansprüche 10 - 14,
dadurch gekennzeichnet,
daß das Abdeckelement (10) des wenigstens einen Einsatzelementes (9) der ersten Werkzeugbacke (1) in der Weise ausgebildet ist, daß zumindest teilweise die obere Oberfläche des Silizium-Chips und damit die druckempfindliche Membranenvorderseite gegenüber Druckbeaufschlagung während des Spritzgußvorganges geschützt ist.

16. Vorrichtung nach einem der Ansprüche 10 - 15,
dadurch gekennzeichnet,
daß der erste bzw. zweite Werkzeugbacken (1 bzw. 2) jeweils mindestens eine Bohrung (7 bzw. 8)-aufweist, in welche das zugeordnete Einsatzelement (9 bzw. 11) von der der Formhöhlung (14) jeweils abgewendeten Oberfläche der Werkzeugbacken her einschraubbar ist, derart, daß das bzw. die jeweilige(n) Abdeckelement(e) (10 bzw. 12, 13) beim gegenseitigen Zusammenfahren der beiden Werkzeugbacken im Bereich der wenigstens einen resultierenden Formhöhlung (14) liegen.

17. Vorrichtung nach einem der Ansprüche 10 - 16,
dadurch gekennzeichnet,
daß die Formhöhlung (14) des Spritzgußwerkzeuges i.w. den Konturen der Spitze (22) eines Katheters (21) entsprechend ausgeformt ist.

18. Vorrichtung nach einem der Ansprüche 10 - 17,
dadurch gekennzeichnet,
daß die Einspritzkanäle bzw. -düsen zum Einspritzen der Spritzgußmasse in die durch die beiden Werkzeugbacken (1, 2) gebildete Formhöhlung im Bereich der einander gegenüberliegenden, im zusammengefahrenen Zustand unmittelbar aneinander angrenzenden, stirnseitigen Oberflächen (5, 6) der Werkzeugbacken angeordnet und ausgebildet sind, derart, daß die Einspritzkanäle bzw. -düsen praktisch im Bereich der Werkzeugbackentrennung bzw. Formtrennung verlaufen.

19. Vorrichtung nach einem der Ansprüche 10 - 18,
dadurch gekennzeichnet,
daß die ersten und zweiten Werkzeugbacken (1, 2) in der Weise relativ zueinander verschiebbar im Spritzgußwerkzeug angeordnet sind, daß die einander gegenüberliegenden stirnseitigen Oberflächen (5, 6) der beiden Werkzeugbacken (1, 2) in vorgegebener Richtung aufeinander zu fahrbar bzw. in entgegengesetzter Richtung voneinander trennbar sind, wobei im aufeinander zugefahrenen Zustand bis zur gegenseitigen Kontaktgabe der beiderseitigen stirnseitigen Oberflächen (5, 6) die erste Werkzeugbacke (1) relativ zur zweiten Werkzeugbacke (2) in einer zur Zusammenfahrrichtung im wesentlichen senkrechten Richtung vorzugsweise um einige Mikrometer verschiebbar ist, derart, daß sich das Abdeckelement (10) in entsprechender Weise in Richtung auf das Abdeckelement (12) hin bewegt.

20. Vorrichtung nach einem der Ansprüche 10 - 19,
dadurch gekennzeichnet,
daß die Einsatzelemente (9, 11) der beiden Werkzeugbacken (1, 2) als erodierte Edelstahl-Einsätze mit hochpolierten Oberflächen im Bereich der Formhöhlung (14) ausgebildet sind.

Einspritzung

Kühlung

Formtrennung

15
4
16
14"
13
12
8
11
6
2

Fig. 1b

3
5
10
14'
7
9
1

Fig. 1a

Fig. 2

EP 0 346 786 A2

Fig.3

Fig.4

Fig. 5

Fig. 6